(19) Europäisches Patentamt
European Patent Office
Office européen des brevets

(11) Veröffentlichungsnummer: **0 007 398**
**A1**

(12) # EUROPÄISCHE PATENTANMELDUNG

(21) Anmeldenummer: 79101656.1

(22) Anmeldetag: 30.05.79

(51) Int. Cl.³: C 07 D 317/46
A 01 N 9/12, A 01 N 9/28
//(C07D317/46, C07C145/04)

(30) Priorität: 08.06.78 CH 6284/78

(43) Veröffentlichungstag der Anmeldung:
06.02.80 Patentblatt 80/3

(84) Benannte Vertragsstaaten:
BE CH DE FR GB IT LU NL SE

(71) Anmelder: F.Hoffmann-La Roche & Co.
Aktiengesellschaft
Abt. VIII-Pt
CH-4002 Basel(CH)

(72) Erfinder: Pfiffner, Albert, Dr.
Grampenweg 10
CH-8180 Bülach(CH)

(74) Vertreter: Lederer, Franz, Dr. et al,
Patentanwälte Dr. Franz Lederer Reiner F. Meyer
Lucile-Grahn-Strasse 22
D-8000 München 80(DE)

(54) **Thio-methylcarbamat-derivat, Verfahren zu seiner Herstellung, seine Verwendung und Pesticide, die es enthalten.**

(57) Die Erfindung betrifft N,N'- Thio-bis (2, 3-isopropylidendioxyphenyl -N-methylcarbamat) der Formel

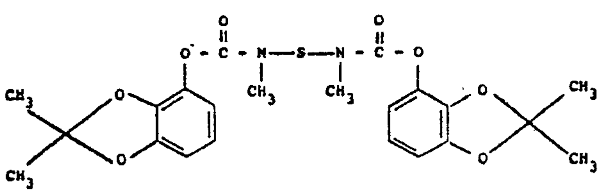

I

welches sich als Schädlingsbekämpfungsmittel, insbesondere zur Bekämpfung von Insekten, eignet, seine Herstellung durch Umsetzung von 2,3-Isopropylidendioxyphenyl-N-methylcarbamat mit Schwefeldichlorid oder Dischwefeldichlorid in Gegenwart einer Base und seine Verwendung.

EP 0 007 398 A1

0007398

30. Mai 1979

BEZEICHNUNG GEÄNDERT
siehe Titelseite

F. Hoffmann-La Roche & Co. Aktiengesellschaft, Basel, Schweiz

RAN 6101/88

Thio-methylcarbamat-derivat, Herstellung dieser Verbindung, sowie Schädlingsbekämpfungsmittel, die diese Verbindung enthalten, und deren Verwendung als Schädlingsbekämpfungsmittel.

Die Erfindung betrifft Bis-[2,3-(isopropylidendioxy)-phenyl]-thio-bis-N-methylcarbamat der Formel

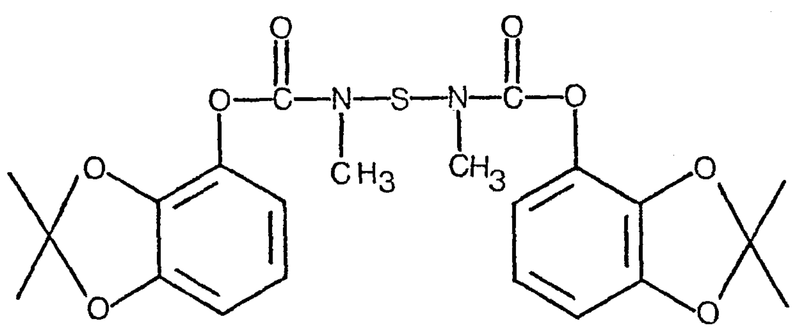

I

Die Verbindung der Formel I ist ein Schädlingsbekämpfungsmittel und eignet sich insbesondere zur Bekämpfung von Insekten. Somit umfasst die Erfindung auch Schädlingsbekämpfungsmittel, welche die Verbindung der Formel I enthalten, Verfahren zur Herstellung derartiger Mittel sowie deren Verwendung.

Hof/19.3.1979

Die Erfindung betrifft ferner Verfahren zur Herstellung der Verbindung der Formel I. Das erfindungsgemässe Verfahren ist dadurch gekennzeichnet, dass man 2,2-Dimethyl-1,3-benzodioxan-4-yl-N-methylcarbamat der Formel

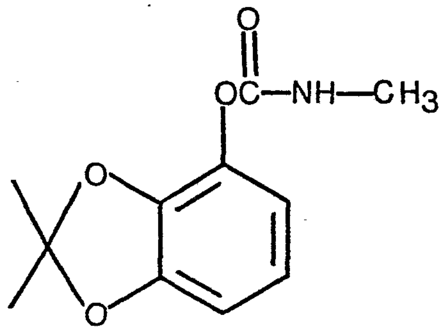

II

mit Schwefeldichlorid oder Dischwefeldichlorid in Gegenwart einer Base umsetzt.

Das Carbamat der Formel II wird entweder mit Schwefeldichlorid oder Dischwefeldichlorid in Gegenwart einer Base wie beispielsweise Triäthylamin oder Pyridin umgesetzt. Als Lösungsmittel für diese Reaktion eignen sich inerte organische Lösungsmittel wie beispielsweise Chloroform oder Methylenchlorid. Die Reaktion wird zweckmässigerweise zwischen Raumtemperatur und der Rückflusstemperatur des Reaktionsgemisches durchgeführt. Die Aufarbeitung erfolgt in üblicher Weise durch Abdestillieren des gewaschenen und getrockneten Lösungsmittels.

Die im erfindungsgemässen Verfahren eingesetzten Ausgangsmaterialien sind bekannt.

Die vorliegende Erfindung betrifft auch pestizide Zusammensetzungen, die als aktiven Bestandteil die Verbindung der Formel I enthalten. Die pestizide Zusammensetzung enthält zweckmässigerweise zumindest eines der folgenden Materialien: Trägerstoffe, Netzmittel, inerte Verdünnungsmittel und Lösungsmittel.

Die vorliegende Erfindung betrifft ferner die Behandlung von Pflanzen, Tieren und des Erdbodens sowie von Gegenständen und Flächen, welche darin besteht, dass auf

diese die Verbindung der Formel I, wie oben definiert, aufgebracht wird.

Die Verbindung der Formel I ist somit ganz allgemein als Pestizid von Wert. Sie zeigt sich besonders wertvoll als Insektizid, insbesondere gegen Coleoptera wie z.B. Rapskäfer, Blattkäfer, Epilachna spp., Leptinotarsa decemlineata, Anthonomus spp., Hypera postica, Conotrachelus nenuphar; gegen Lepidoptera wie z.B. Laspeyresia spp., Heliothis spp.; Adoxophyes orana gegen Heteroptera wie z.B. Lygus spp. und Dysdercus cingulatus; gegen Diptera wie z.B. Aedes aegypti.

Die Verbindung wirkt als direktes Insektizid. Sie hat auch systemische Aktivität. Sie zeichnet sich unter Praxisbedingungen durch eine besonders günstige Wirkungsdauer aus. Die Verbindung ist ferner von Wert für die Bekämpfung von Schädlingen bei Tieren. So zeigt beispielsweise die Verbindung der Formel I in einer Konzentration von $10^{-7}$ g/ml im Test gegen Aedes aegypti eine Wirkung von 100%. Die akute orale Toxizität an der Maus beträgt mehr als 1000 mg/kg.

Ausserdem zeichnet sich die erfindungsgemässe Verbindung der Formel I durch eine hohe UV-Stabilität aus.

Die Verbindung der Formel I ist wasserunlöslich und kann nach irgendeiner der für unlösliche Verbindungen üblichen Methoden konfektioniert werden.

Wenn gewünscht, kann die Verbindung der Formel I in einem mit Wasser nicht mischbaren Lösungsmittel, wie beispielsweise einem hochsiedenden Kohlenwasserstoff, gelöst werden, das zweckmässigerweise gelöste Emulgatoren enthält, so dass es bei Zugabe zu Wasser als selbstemulgierbares Oel wirkt.

Die Verbindung der Formel I kann auch mit einem Netzmittel mit oder ohne inertes Verdünnungsmittel zur Bildung eines netzbaren Pulvers vermischt werden, welches in Wasser

löslich oder dispergierbar ist, oder sie kann mit dem inerten Verdünnungsmittel zur Bildung eines festen oder pulverförmigen Produktes vermischt werden.

Inerte Verdünnungsmittel, mit welchen die Verbindung der Formel I verarbeitet werden kann, sind feste inerte Medien, einschliesslich pulverförmige oder feinverteilte Feststoffe, wie beispielsweise Tone, Sande, Talk, Glimmer, Düngemittel und dgl., wobei solche Produkte entweder staubförmig oder als Materialien mit grösserer Teilchengrösse vorliegen können.

Die Netzmittel können anionische Verbindungen, wie beispielsweise Seifen, Fettsulfatester, wie Dodecylnatriumsulfat, Octadecylnatriumsulfat und Cetylnatriumsulfat, fettaromatische Sulfonate, wie Alkylbenzolsulfonate oder Butylnaphthalinsulfonate, komplexerer Fettsulfonate, wie die Amidkondensationsprodukte von Oelsäure und N-Methyltaurin oder das Natriumsulfonat von Dioctylsuccinat sein.

Die Netzmittel können auch nichtionische Netzmittel, wie beispielsweise Kondensationsprodukte von Fettsäuren, Fettalkoholen oder fettsubstituierten Phenolen mit Aethylenoxyd, oder Fettsäureester und -äther von Zuckern oder mehrwertigen Alkoholen oder die Produkte, die aus letzteren durch Kondensation mit Aethylenoxyd erhalten werden, oder die Produkte, die als Blockcopolymere von Aethylenoxyd und Propylenoxyd bekannt sind, sein. Die Netzmittel können auch kationische Mittel, wie beispielsweise Cetyltrimethylammoniumbromid und dgl. sein.

Die pestizide Zusammensetzung kann auch in Form einer Aerosolverbindung vorliegen, wobei zweckmässigerweise zusätzlich zum Treibgas, welches geeigneterweise ein polyhalogeniertes Alkan, wie Dichlordifluormethan ist, ein Co-Solvens und ein Netzmittel verwendet wird.

Die pestiziden Zusammensetzungen gemäss der vorliegenden Erfindung können zusätzlich zu der Verbindung der Formel I andere aktive Insektizide, Bakterizide und Fungizide enthalten.

In ihren verschiedenen Anwendungsgebieten kann die erfindungsgemässe Verbindung in unterschiedlichen Mengenverhältnissen eingesetzt werden; so werden beispielsweise für die Behandlung von Pflanzen zur Bekämpfung von Schädlingen auf diesen die Verbindung zweckmässigerweise in einem Ausmass von etwa 100-2000 g/ha verwendet, während für die Bodenapplikation höhere Mengen zur Anwendung gelangen.

Die folgenden Beispiele sollen die vorliegende Erfindung illustrieren.

Beispiel 1

Eine Lösung von 66,9 g 2,2-Dimethyl-1,3-benzodioxan-4-yl-N-methylcarbamat (BENDIOCARB), 37,8 g Pyridin in 210 ml Methylenchlorid werden unter Rückfluss tropfenweise mit 18,75 g Schwefeldichlorid versetzt und eine halbe Stunde am Rückfluss gehalten. Die erhaltene Reaktionslösung wird mit Wasser gewaschen, über Natriumsulfat getrocknet und eingedampft. Durch Filtration an der zehnfachen Menge Kieselgel mit Methylenchlorid als Lösungsmittel und anschliessender Umkristallisation aus Isopropanol wird reines Bis-[2,3-(isopropylidendioxy)-phenyl]-thio-bis-N-methylcarbamat erhalten; Smp. 138-138,5°C.

Beispiel 2

Die Wirkstoffe können für die biologische Prüfung wie folgt formuliert werden:

| | % w/w |
|---|---|
| Wirkstoff | 50,0 |
| Silcasil S (Kieselsäure, hydratisiert, ca. 87% $SiO_2$) | 5,0 |
| Natrium-laurylsulfat | 1,0 |
| Natrium-lignosulfonat | 2,0 |
| Kaolin B 24 (hauptsächlich $Al_2Si_2O_5(OH)_4$) | 42,0 |

Beispiel 3

Testinsekt: Epilachna chrysomelina, Gurkenkäfer
Testmethode: Persistenztest

Kürbispflanzen werden bis zum Abtropfen behandelt. Nach dem Antrocknen werden die Pflanzen in einem Ecophyten bei 30°C und UV-Bestrahlung während 7 Tagen inkubiert. Nach der Inkubation werden pro Pflanze 10 Larven des ersten Stadiums angesetzt. Die Resultate werden ausgedrückt als prozentuale Reduktion der Ueberlebensrate der Epilachna-Larven im Vergleich zu den Kontrollparzellen.

| Wirkstoff | Konz. % ai | % Wirkung |
|---|---|---|
| Formel I | 0,03 | 100 |
| | 0,01 | 100 |
| Formel II | 0,03 | 100 |
| | 0,01 | 30 |

Die Formulierung der Wirkstoffe erfolgte gemäss Beispiel 2.

ai = Wirkstoff

## Beispiel 4

Testinsekt: Leptinotarsa decemlineata, Kartoffelkäfer
Testmethode: Persistenztest

Kartoffelpflanzen werden bis zum Abtropfen behandelt. Nach dem Antrocknen werden die Pflanzen in einem Ecophyten bei 30°C und UV-Bestrahlung während 7 Tagen inkubiert. Nach der Inkubation werden pro Pflanze 10 Larven des ersten Stadiums angesetzt. Die Resultate werden ausgedrückt als prozentuale Reduktion der Ueberlebensrate der Leptinotarsa-Larven im Vergleich zu den Kontrollparzellen.

| Wirkstoff | Konz. % ai | % Wirkung |
|---|---|---|
| Formel I | 0,03 | 100 |
| | 0,01 | 100 |
| Formel II | 0,03 | 95 |
| | 0,01 | 5 |

Die Formulierung der Wirkstoffe erfolgte gemäss Beispiel 2

ai = Wirkstoff

## Beispiel 5

Testinsekt: Leptinotarsa decemlineata, Kartoffelkäfer
Testmethode: Initialwirkung auf Blattrondellen

Kartoffelblattrondellen werden mit acetonischer Wirkstofflösung besprüht. Pro Rondelle werden 5 frischgeschlüpfte Larven des ersten Stadiums angesetzt und bei 25°C und 60% rel. Feuchtigkeit inkubiert. Unbehandelte und mit Aceton behandelte Blattrondellen dienen als Kontrolle. Testdauer: 4 Tage. Die Resultate werden ausgedrückt als prozentuale Reduktion der Ueberlebensrate der Larven im Vergleich zu den Kontrollparzellen.

| Wirkstoff | Dosis ($10^{-x}$g ai/$cm^2$) | % Wirkung |
|---|---|---|
| Formel I | 7 | 100 |
| | 8 | 79 |
| | 9 | 0 |
| Formel II | 7 | 100 |
| | 8 | 8 |
| | 9 | 0 |

ai = Wirkstoff

## Beispiel 6

Testinsekt: Aedes aegypti, Gelbfiebermücke
Testmethode: Initialwirkung im Wasser

0,2 ml acetonische Wirkstofflösung werden mit 20 ml Leitungswasser gemischt. Pro Parzelle werden 10 Larven im letzten Larvenstadium angesetzt und bei 25°C und 60% rel. Feuchtigkeit inkubiert bis zum Schlüpfen der Adulten in den Kontrollparzellen. Unbehandelte und mit Aceton behandelte Parzellen dienen als Kontrollen. Testdauer: 7 Tage. Die Wirkung wird ausgedrückt als prozentuale Reduktion der Zahl normaler Adulten im Vergleich zu den Kontrollparzellen.

| Wirkstoff | Dosis ($10^{-x}$g ai/ml Wasser) | % Wirkung |
|---|---|---|
| Formel I | 6 | 100 |
| | 7 | 100 |
| | 8 | 13 |
| Formel II | 6 | 100 |
| | 7 | 2 |
| | 8 | 0 |

ai = Wirkstoff

## Patentansprüche

1. Bis-[2,3-(isopropylidendioxy)-phenyl]-thio-bis-N-methylcarbamat.

2. Bis-[2,3-(isopropylidendioxy)-phenyl]-thio-bis-N-methylcarbamat als Schädlingsbekämpfungsmittel.

3. Schädlingsbekämpfungsmittel, dadurch gekennzeichnet, dass es Bis-[2,3-(isopropylidendioxy)-phenyl]-thio-bis-N-methylcarbamat und inertes Trägermaterial enthält.

4. Verfahren zur Herstellung von Bis-[2,3-(isopropylidendioxy)-phenyl]-thio-bis-N-methylcarbamat der Formel

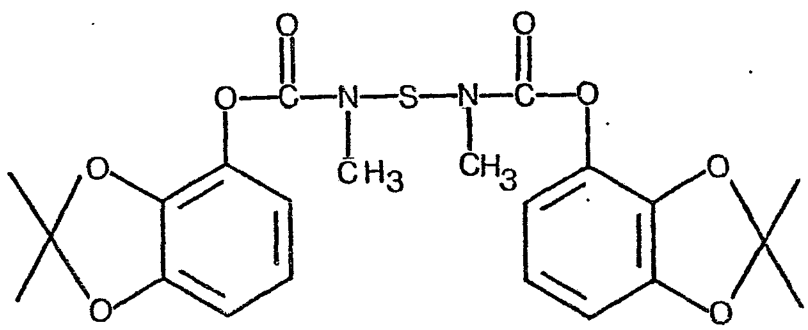

I

dadurch gekennzeichnet, dass man 2,3-Dimethyl-1,3-benzodioxan-4-yl-N-methylcarbamat der Formel

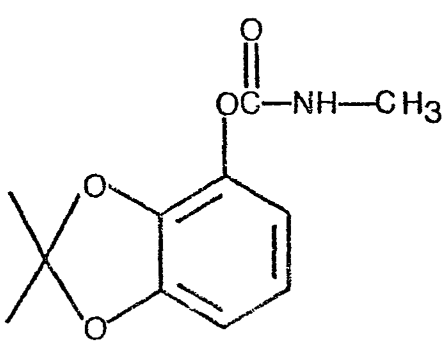

II

mit Schwefeldichlorid oder Dischwefeldichlorid in Gegenwart einer Base umsetzt.

5. Verfahren zur Bekämpfung von Schädlingen, dadurch gekennzeichnet, dass man die zu schützenden Gegenstände mit Bis-[2,3-(isopropylidendioxy)-phenyl]-thio-bis-N-methylcarbamat behandelt.

6. Verwendung von Bis-[2,3-(isopropylidendioxy)-phenyl]-thio-bis-N-methylcarbamat als Schädlingsbekämpfungsmittel.

***

Europäisches Patentamt

# EUROPÄISCHER RECHERCHENBERICHT

Nummer der Anmeldung
79101656

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | DE-A-2 131 399 (BASF) * S.2/3 unter a)* | 4 |
| | US-A 3 679 733 (BROWN) * Spalte 2 * | 4 |
| | DE-A-2 611 042 (F.HOFFMANN-LA ROCHE) * S.6, Ansprüche 4-11* | 3,5 |
| | DE-A-2 607 655 (F.HOFFMANN-LA ROCHE) * S.3, Ansprüche 2-5 * | 3,5 |
| | DE-A-2 600 981 (BAYER) * S.42, Nr.33 und S.19 * | 3,5 |
| | DE-A-2 428 924 (BAYER) * S.15/16, Nr.1-3, S.12 und 14* | 3,5 |
| | DE-A-2 254 359 (BAYER) *S.11,13,21 (vor) letzte Verb.* | 3,5 |
| | DE-A-2 221 706 (FISONS) *S.2 unter (a), S.9* | 4 |
| | DE-A-2 738 361 (F.HOFFMANN-LA ROCHE) * Beispiele 1-11* | 3,5 |
| | DE-A-2 341 949 (FISONS) *Beispiele 5-11* | 3,5 |

KLASSIFIKATION DER ANMELDUNG (Int.Cl.³)

C07D 317/46
A01N 9/12
A01N 9/28 //
(C07D 317/46, C07C 145/04)

RECHERCHIERTE SACHGEBIETE (Int. Cl.³)

C07D 317/00,08, 44,46
C07C 149/437
C07C 145/00,04
A01N 9/12
A 01N 9/28

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Wien | 25.Juli 1979 | Daschl |